# FASCICULE DE BREVET EUROPEEN

(11) **EP 2 403 578 B1**
(45) Date de publication et mention de la délivrance du brevet: **13.11.2019**
(21) Numéro de dépôt: 10715283.7
(22) Date de dépôt: 01.03.2010
(51) Int. Cl.: A61M 25/00, A61B 17/435, A61D 19/04

(54) **CATHÉTER POUR L'IMPLANTATION D'EMBRYONS DANS UNE CAVITÉ UTÉRINE D'UN ÊTRE HUMAIN OU ANIMAL, ET INSTRUMENT CORRESPONDANT.**
KATHETER ZUR IMPLANTIERUNG EINES EMBRYOS IN DER UTERUSHÖHLE EINES MENSCHEN ODER TIERS UND ENTSPRECHENDES INSTRUMENT
CATHETER FOR IMPLANTING AN EMBRYO IN THE UTERINE CAVITY OF A HUMAN BEING OR ANIMAL, AND CORRESPONDING INSTRUMENT

(30) Priorité: 04.03.2009 FR 0951354
(43) Date de publication de la demande: 11.01.2012
(73) Titulaire: Assistance Publique - Hôpitaux de Paris, 75001 Paris (FR); Université Paris Descartes, 75270 Paris Cedex 06 (FR); Université Paris 13, 93430 Villetaneuse (FR)
(72) Inventeur: WOLF, Jean-Philippe, 75013 Paris (FR); PONCELET, Christophe, 95150 Taverny (FR)
(74) Mandataire: Lavoix
(86) Numéro de dépôt international: PCT/FR2010/050346
(87) Numéro de publication internationale: WO 2010/100373

(56) Documents cités:
- EP-A- 0 478 155
- WO-A1-99/37348
- DE-A1- 10 240 279
- FR-A- 2 524 324
- FR-A- 2 715 824
- US-A- 4 642 094

## Description

La présente invention concerne un cathéter pour l'implantation d'embryons dans une cavité utérine d'un être humain ou animal, du type comprenant un corps de cathéter tubulaire définissant une lumière longitudinale.

Des exemples de cathéters de l'état de la technique sont décrits dans WO 99/37348, FR 2 524 324, DE 102 40 279 et US 4 642 094.

Le transfert embryonnaire reste un geste aveugle et non physiologique. Il est en effet pratiqué par voie transcervicale à l'aide d'un cathéter du type précité, qui est introduit par le canal endocervical pour pénétrer dans la cavité utérine, laquelle est tapissée d'un endomètre au maximum de son développement. Il n'est donc pas impossible que cette opération puisse traumatiser la muqueuse endométriale et compromettre les chances de succès du transfert.

Les lésions endométriales causées par les cathéters actuels peuvent donc gêner l'implantation d'un embryon si celui-ci est déposé à la surface de la nappe sanguine, ce qui est le cas avec un cathéter classique.

L'invention a pour but de proposer un cathéter qui permette d'améliorer les conditions de l'interface embryon/endomètre de manière à favoriser l'implantation embryonnaire.

A cet effet, l'invention a pour objet un cathéter selon la revendication 1.

Le cathéter selon l'invention peut comporter une ou plusieurs des caractéristiques suivantes :
- le cathéter comprend, au voisinage de l'extrémité proximale du corps de cathéter, un repère de la position angulaire de l'orifice ;
- l'extrémité distale du corps de cathéter présente une forme arrondie dépourvue d'arête vive ;
- le cathéter comprend un embout fixé sur l'extrémité proximale du corps de cathéter et adapté pour recevoir des moyens d'injection ;
- le cathéter comprend un insert métallique disposé au voisinage de l'extrémité distale du corps de cathéter ;
- le corps de cathéter est fabriqué à partir d'une matière plastique souple adaptée pour pouvoir être coudée manuellement ;
- le tronçon d'extrémité distale courbe est sensiblement en arc de cercle ; et
- le corps de cathéter est fabriqué à partir d'une matière hydrophobe

L'invention a également pour objet un instrument, notamment pour l'implantation d'embryons dans une cavité utérine d'un être humain ou animal, du type comprenant un cathéter et des moyens d'injection raccordables à l'extrémité proximale du cathéter, caractérisé en ce que le cathéter est tel que décrit précédemment.

L'invention sera mieux comprise à la lecture de la description qui va suivre, donnée uniquement à titre d'exemple et faite en se référant aux dessins annexés, sur lesquels :
- la Figure 1 est une vue en coupe frontale d'un utérus ;
- la Figure 2 est une vue en coupe longitudinale d'un instrument de transfert embryonnaire muni d'un cathéter selon l'invention ;
- la Figure 3 est une vue agrandie du détail indiqué III sur la Figure 2 ; et
- la Figure 4 est une vue en coupe latérale de l'utérus de la Figure 1 dans lequel est introduit l'instrument de la Figure 2.

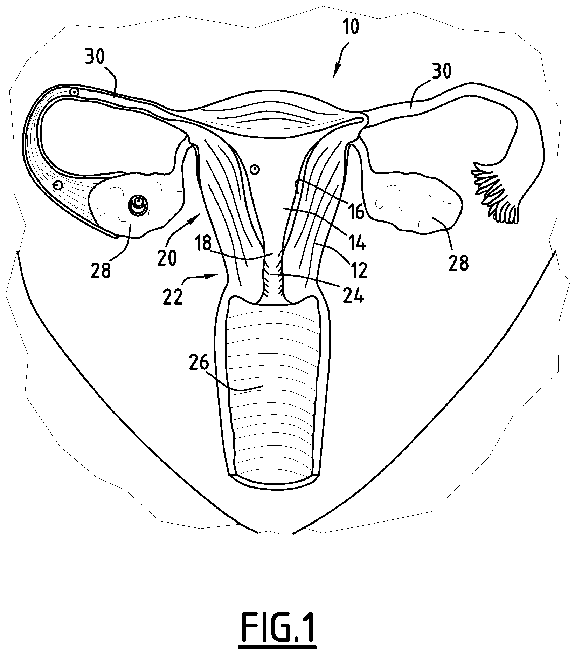

Afin de mieux comprendre la description qui va suivre, quelques rappels d'anatomie féminine sont données en référence à la Figure 1, sur laquelle est représenté un utérus 10.

L'utérus 10 est un organe musculaire creux destiné à contenir un œuf fécondé pendant son développement et à l'expulser quand il est arrivé à maturité. Autrement dit, c'est un organe dans lequel se développe un embryon, puis le fœtus correspondant, et qui expulse ce dernier à l'accouchement.

Le muscle formant l'utérus 10 est appelé le myomètre 12, la cavité qui se trouve à l'intérieur de ce muscle est appelée la cavité utérine 14 et la muqueuse qui tapisse la cavité utérine 14 est appelée l'endomètre 16.

L'utérus 10 a la forme d'un cône à sommet tronqué dirigé vers le bas. Entre la base et le sommet de l'utérus 10, un léger étranglement nommé l'isthme utérin 18 sépare l'utérus en deux parties : une partie supérieure nommée le corps utérin 20 et une partie inférieure nommée le col utérin 22.

Le col utérin 22 est percé en son milieu par un canal appelé le canal endocervical 24 qui relie la cavité utérine 14 à la cavité vaginale 26.

L'utérus 10 est relié, de chaque côté, à un ovaire 28 par une trompe de Fallope 30.

Un transfert embryonnaire consiste à déposer délicatement un ou plusieurs embryons fécondés en laboratoire, par exemple par une technique de fécondation in vitro (FIV) classique ou d'ICSI (Intra Cytoplasmic Sperm Injection), dans la cavité utérine 14 à l'aide d'un cathéter.

Si le développement des embryons se poursuit normalement, ils vont s'implanter dans l'endomètre 16 à condition que celui-ci soit adéquat.

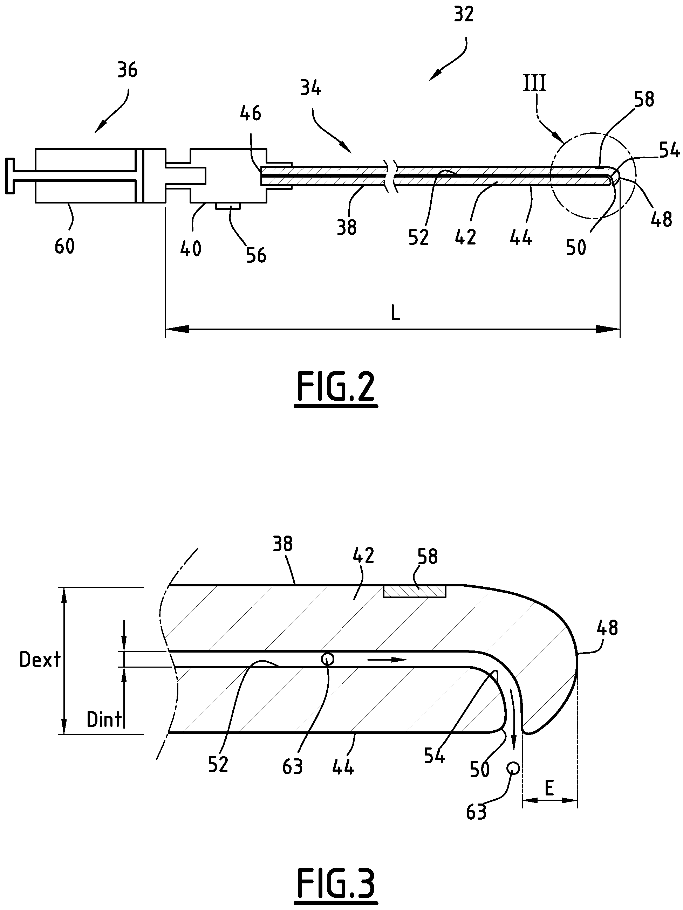

La Figure 2 illustre, de façon schématique, un instrument 32 de transfert embryonnaire conforme à l'invention.

L'instrument 32 comprend un cathéter 34 et des moyens d'injection 36 raccordables au cathéter 34.

Le cathéter 34 comprend un corps de cathéter 38 et un embout 40 fixé à l'extrémité proximale du corps 38.

Le corps de cathéter 38 possède une forme tubulaire cylindrique définie par une paroi latérale 42 cylindrique présentant une surface externe 44, et possède une extrémité proximale 46 et une extrémité distale 48.

Comme on le voit mieux sur la Figure 3, un orifice latéral 50 unique est ménagé dans la paroi latérale 42, à une distance E de l'extrémité distale 48 sensiblement égale à 2 mm.

Le corps de cathéter 38 délimite une lumière interne 52 longitudinale qui s'étend depuis l'extrémité proximale 46 jusqu'au voisinage de l'extrémité distale 48.

La lumière 52 est sensiblement rectiligne et comporte un tronçon 54 d'extrémité distale courbe, sensiblement en quart de cercle.

Le tronçon 54 débouche vers l'extérieur du corps de cathéter 38 par l'orifice 50, et sensiblement perpendiculairement à l'axe du cathéter 34 qui correspond à l'axe de la lumière 52.

La lumière 52 présente un diamètre Dint sensiblement constant sur toute sa longueur et compris entre 150 µm (valeur correspondant sensiblement à la taille d'un embryon) et 200 µm.

Ainsi, si plusieurs embryons sont prélevés, ils seront nécessairement disposés dans la lumière 52 les uns à la suite des autres.

En variante ne formant pas partie de l'invention, dans le cas où l'instrument est utilisé pour le transfert de blastocyste, on prévoit une lumière 52 présentant un diamètre Dint sensiblement constant sur toute sa longueur et compris entre 200 µm (valeur correspondant sensiblement à la taille d'un blastocyste) et 250 µm.

L'extrémité distale 48 du corps de cathéter 38 est fermée et présente une forme arrondie, lisse, dépourvue d'arête vive et d'aspérité, sensiblement en demi-sphère.

Le corps de cathéter 38 présente un diamètre externe Dext sensiblement égal à 1,5 mm et est fabriqué à partir d'une matière plastique souple pour pouvoir être coudée manuellement par un praticien de façon à s'adapter à l'anatomie de la patiente.

Bien entendu, la matière plastique utilisée est également biocompatible avec l'organisme humain.

De préférence, la matière utilisée pour fabriquer le corps de cathéter 38 est hydrophobe, afin de limiter les risques d'accolement de l'embryon dans la lumière 52.

L'embout 40 est fixé de façon étanche sur l'extrémité proximale 46 du corps de cathéter 38 et reçoit les moyens d'injection 36.

Un repère 56 de la position angulaire de l'orifice 50 (Figure 2), par exemple une ailette, est indiqué sur l'embout 40.

Le cathéter 34 comprend un insert 58 métallique d'écho-guidage disposé à l'intérieur de la paroi latérale 42 du corps de cathéter 38, à proximité de l'extrémité distale 48.

Le cathéter 34 présente, corps de cathéter 38 et embout 40 compris, une longueur L de l'ordre d'une vingtaine de centimètres, de préférence inférieure à 22 cm.

Les moyens d'injection 36 comprennent une seringue 60 raccordée de façon étanche à l'embout 40 et ayant une capacité de 1 ml.

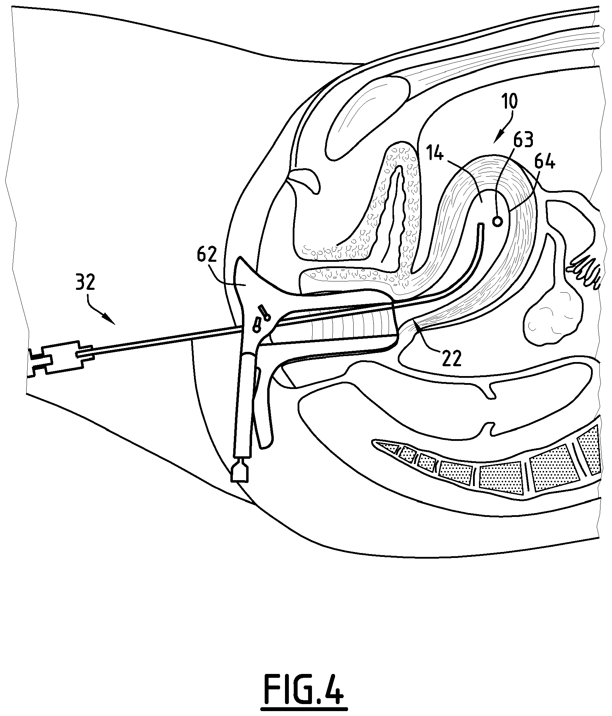

Un protocole de transfert embryonnaire utilisant l'instrument 32 va maintenant être décrit en référence à la Figure 4.

La patiente est installée en position gynécologique.

Le praticien pose un spéculum 62 de manière à visualiser le col utérin 22 et nettoie ce dernier.

A l'aide d'une sonde échographique posée sur le ventre, le praticien repère l'utérus 10.

En fonction de l'anatomie de la patiente, le praticien peut couder légèrement ou non la partie d'extrémité distale du cathéter 34 de manière à faciliter l'introduction de l'instrument 32 dans l'utérus 10.

Le praticien passe alors l'instrument 32, contenant le ou les embryons 63 dans quelques microlitres de milieu de culture, au travers du col utérin 22 de manière à ce que l'extrémité distale 48 soit positionnée dans la cavité utérine 14.

Le nombre d'embryons à transférer est généralement de un, de manière à éviter les grossesses multiples.

L'avancée de l'instrument 32 dans l'utérus 10 est réalisée sous contrôle échographique à l'aide de la sonde et repérable grâce à l'insert métallique 58.

Une fois que l'extrémité distale 48 atteint presque le fond de la cavité utérine 14, le praticien oriente l'orifice 50 à l'aide du repère 56 en direction de la paroi postéro fundique 64 de la cavité utérine 14, qui semble être le lieu idéal pour le développement des embryons.

En variante, le praticien peut décider de déposer le ou les embryons dans toute autre localisation qui lui paraît plus indiquée.

Il suffit alors au praticien d'actionner la seringue 60 afin d'expulser les embryons.

Enfin, le praticien retire lentement l'instrument 32 de l'utérus 10.

L'invention propose donc un instrument de transfert embryonnaire qui permet d'améliorer le succès de l'assistance médicale à la procréation en déposant les embryons à distance du trajet du cathéter et non dans son axe.

Le positionnement latéral de l'orifice unique à distance de l'extrémité distale permet non seulement d'éloigner les embryons des éventuelles lésions endométriales générées par l'introduction du cathéter dans l'utérus, mais également d'expulser sélectivement les embryons vers une zone idéale d'implantation.

De plus, la forme arrondie et lisse de l'extrémité distale du cathéter permet de rendre l'introduction de l'instrument moins traumatisante pour l'endomètre.

Les embryons utilisés peuvent être des embryons issus d'une fécondation in vitro, d'une fécondation par ICSI ou encore des embryons congelés.

L'instrument selon l'invention peut également avoir un usage vétérinaire en s'appliquant au transfert embryonnaire dans la cavité utérine d'un animal femelle.

## Revendications

1. Cathéter (34) pour l'implantation d'embryons (63) dans une cavité utérine (14) d'un être humain ou animal, du type comprenant un corps de cathéter (38) tubulaire définissant une lumière (52) longitudinale, dans lequel :
- l'extrémité distale (48) du corps de cathéter (38) est fermée,
- la lumière (52) débouche par un orifice latéral unique (50) sur une surface externe (44) d'une paroi latérale (42) du corps de cathéter (38), à distance de cette extrémité distale (48),
- la lumière (52) comporte un tronçon (54) d'extrémité distale courbe, et
- la lumière (52) possède un diamètre (Dint) sensiblement constant sur toute sa longueur,
**caractérisé en ce que** le diamètre (Dint) de la lumière (52) est sensiblement supérieur à 150 µm et inférieur à 200 µm.

2. Cathéter (34) selon la revendication 1, **caractérisé en ce qu'**il comprend, au voisinage de l'extrémité proximale (46) du corps de cathéter (38), un repère (56) de la position angulaire de l'orifice (50).

3. Cathéter (34) selon la revendication 1 ou 2, **caractérisé en ce que** l'extrémité distale (48) du corps de cathéter (38) présente une forme arrondie dépourvue d'arête vive.

4. Cathéter (34) selon l'une quelconque des revendications 1 à 3, **caractérisé en ce qu'**il comprend un embout (40) fixé sur l'extrémité proximale (46) du corps de cathéter (38) et adapté pour recevoir des moyens d'injection (36).

5. Cathéter (34) selon l'une quelconque des revendications 1 à 4, **caractérisé en ce qu'**il comprend un insert métallique (58) disposé au voisinage de l'extrémité distale (48) du corps de cathéter (38).

6. Cathéter (34) selon l'une quelconque des revendications 1 à 5, **caractérisé en ce que** le corps de cathéter (38) est fabriqué à partir d'une matière plastique souple adaptée pour pouvoir être coudée manuellement.

7. Cathéter (34) selon l'une quelconque des revendications 1 à 6, **caractérisé en ce que** le tronçon (54) d'extrémité distale courbe est sensiblement en arc de cercle.

8. Cathéter (34) selon l'une quelconque des revendications 1 à 7, **caractérisé en ce que** le corps de cathéter (38) est fabriqué à partir d'une matière hydrophobe.

9. Instrument (32) pour l'implantation d'embryons (63) dans une cavité utérine (14) d'un être humain ou animal, du type comprenant un cathéter (34) et des moyens d'injection (36) raccordables à l'extrémité proximale (46) du cathéter (34), **caractérisé en ce que** le cathéter (34) est conforme à l'une quelconque des revendications précédentes.

## Patentansprüche

1. Katheter (34) für die Implantierung von Embryonen (63) in einen Uterus (14) eines Menschen oder Tieres des Typs, der einen rohrförmigen Katheterkörper (38) aufweist, der ein langgestrecktes Lumen (52) definiert, in dem:
- das distale Ende (48) des Katheterkörpers (38) geschlossen ist,
- das Lumen (52) über eine einzige seitliche Öffnung (50) an einer Außenfläche (44) einer Seitenwand (42) des Katheterkörpers (38) mit Abstand zu diesem distalen Ende mündet,
- das Lumen (52) einen gekrümmten Abschnitt(54) des distalen Endes aufweist,
- das Lumen (52) einen im Wesentlichen konstanten Durchmesser (Dint) über seine gesamte Länge besitzt,
**dadurch gekennzeichnet, dass** der Durchmesser (Dint) des Lumens (52) merkbar größer als 150 µm und kleiner als 200 µm ist.

2. Katheter (34) nach Anspruch 1, **dadurch gekennzeichnet, dass** er in der Nähe des proximalen Endes (46) des Katheterkörpers (38) eine Koordinatenreferenz (56) der Winkelposition der Öffnung (50) enthält.

3. Katheter (34) nach Anspruch 1 oder 2, **dadurch gekennzeichnet, dass** das distale Ende (48) des Katheterkörpers (38) eine abgerundete Form ohne merkbare Kante aufweist,

4. Katheter (34) nach einem beliebigen der Ansprüche 1 bis 3, **dadurch gekennzeichnet, dass** er einen an dem proximalen Ende (46) des Katheterkörpers (38) befestigten Ansatz (40) umfasst, der angepasst ist, Injektionsmittel (36) aufzunehmen.

5. Katheter (34) nach einem beliebigen der Ansprüche 1 bis 4, **dadurch gekennzeichnet, dass** er einen metallischen Einsatz (58) umfasst, der in der Nähe des distalen Endes (48) des Katheterkörpers (38) angeordnet ist.

6. Katheter (34) nach einem beliebigen der Ansprüche 1 bis 5, **dadurch gekennzeichnet, dass** der Katheterkörper (38) aus einem biegbaren Kunststoff hergestellt ist, der manuell gebogen werden kann.

7. Katheter (34) nach einem beliebigen der Ansprüche 1 bis 6, **dadurch gekennzeichnet, dass** der gekrümmte Abschnitt (54) des distalen Endes im Wesentlichen kreisbogenförmig ist.

8. Katheter (34) nach einem beliebigen der Ansprüche 1 bis 7, **dadurch gekennzeichnet, dass** der Katheterkörper (38) aus einem hydrophoben Material hergestellt ist.

9. Instrument (32) für die Implantierung von Embryonen in einen Uterus (14) eines Menschen oder eines Tieres des Typs, der einen Katheter (34) und Injektionsmittel (36), die an das proximale Ende (46) des Katheters (34) anschließbar sind, umfasst, **dadurch gekennzeichnet, dass** der Katheter (34) nach einem beliebigen der vorhergehenden Ansprüche ausgebildet ist.

## Claims

1. A catheter (34) for implanting embryos (63) in a uterine cavity (14) of a human being or animal, of the type comprising a tubular catheter body (38) defining a longitudinal channel (52), wherein:
- the distal end (48) of the cavity body (38) is closed,
- the channel (52) opens out through a single side orifice (50) on an external surface (44) of a side wall (42) of the catheter body (38), at a distance from this distal end (48),
- the channel (52) includes a curved distal end segment (54), and
- the channel (52) has a substantially constant diameter (Dint) over the whole of its length,
**characterized in that** the diameter (Dint) of the channel (52) is substantially greater than 150 µm and less than 200 µm.

2. The catheter (34) according to claim 1, **characterized in that** it comprises in the vicinity of the proximal end (46) of the catheter body (38), a mark (56) of the angular position of the orifice (50).

3. The catheter (34) according to claim 1 or 2, **characterized in that** the distal end (48) of the catheter body (38) has a rounded shape without any sharp edge.

4. The catheter (34) according to any of claims 1 to 3, **characterized in that** it comprises an endpiece (40) attached on the proximal end (46) of the catheter body (38) and adapted for receiving injection means (36).

5. The catheter (34) according to any of claims 1 to 4, **characterized in that** it comprises a metal insert (58) positioned in the vicinity of the distal end (48) of the catheter body (38).

6. The catheter (34) according to any of claims 1 to 5, **characterized in that** the catheter body (38) is made from a flexible plastic material adapted so as to be able to be bent manually.

7. The catheter (34) according to any of claims 1 to 6, **characterized in that** the curved distal end segment (54) is substantially as a circular arc.

8. The catheter (34) according to any of claims 1 to 7, **characterized in that** the catheter body (38) is made from a hydrophobic material.

9. An instrument (32) for implanting embryos (63) in a uterine cavity (14) of a human being or animal, of the type comprising a catheter (34) and injection means (36) which may be connected to the proximal end (46) of the catheter (34), **characterized in that** the catheter (34) is in accordance with any of the preceding claims.
